Europäisches Patentamt

**European Patent Office**

Office européen des brevets

(11) Publication number: **0 039 988**

**B1**

# (12) EUROPEAN PATENT SPECIFICATION

(45) Date of publication of patent specification: **23.05.84**

(51) Int. Cl.³: **A 61 N 1/42**

(21) Application number: **81301291.1**

(22) Date of filing: **25.03.81**

(54) **Apparatus for treating an intact animal organism bearing a neoplastic process and undergoing drug treatment.**

(30) Priority: **29.04.80 US 145012**

(43) Date of publication of application:
**18.11.81 Bulletin 81/46**

(45) Publication of the grant of the patent:
**23.05.84 Bulletin 84/21**

(84) Designated Contracting States:
**BE DE FR GB NL**

(56) References cited:
**DE-A-2 353 959**
**FR-A- 716 954**
**FR-A- 788 821**
**FR-A- 948 226**
**FR-A-1 541 165**
**FR-A-2 290 224**
**FR-A-2 362 640**
**FR-A-2 458 277**
**US-A-4 106 488**

(73) Proprietor: **ELECTRO-BIOLOGY, INC**
**300 Fairfield Road**
**Fairfield New Jersey 07006 (US)**

(72) Inventor: **Norton, Larry**
**1601 Third Avenue**
**New York New York 10028 (US)**
Inventor: **Pilla, Arthur A.**
**371 Martom Road**
**Wyckoff New Jersey 07481 (US)**

(74) Representative: **Wright, Peter David John et al**
**R.G.C. Jenkins & Co. 12-15, Fetter Lane**
**London EC4A 1PL (GB)**

Courier Press, Leamington Spa, England.

## Description

This invention relates to apparatus for the treatment of an intact animal organism bearing a neoplastic process and undergoing drug treatment, comprising means of non-paramagnetic material for receiving the animal organism, means providing a magnetic circuit, the providing means being carried by the receiving means and including a multi-turn electrical coil, and means for generating electrical pulses for exciting the coil to generate a predetermined varying magnetic field whereby, in use, to generate, through electro-magnetic, an electrochemical response in the cells and/or tissues of the animal organism.

Such an apparatus is known from FR—A—2 290 224.

In particular, the invention pertains to apparatus for providing controlled modification of cell and/or tissue behaviour during such treatment, by the application of electrical information which may be regarded as encoded. More particularly, the invention provides for the intracellular application, via surgically non-invasive direct inductive coupling, of one or more electrical voltage and concomitant current signals which modify a fundamental behavioural function of the cells and/or tissues, in the context of drug treatment of a cancer affliction.

Attempts have been made in the past to elicit a response of living cells and/or tissue to electrical signals. Much of this work is reviewed and discussed in Ryaby, et al. U.S. Patent No. 4,105,017, particularly insofar as such responses have been elicited in osteogenetic cell and/or tissue behaviour. Reference is made to said patent for extensive background discussion.

Reference is also made to the aforementioned French Patent No. 2,290,224, which discloses apparatus for treating an organism by electromagnetic pulses. Such apparatus includes means for receiving a portion of the organism, an induction coil, and means for generating pulse surges, having a frequency between 0 and 1,000 Hz, to excite the coil.

We have discovered that certain beneficial modifications of cell and/or tissue behaviour may be specifically and selectively induced in an intact animal organism bearing a neoplastic process via electromagnetic coupling using the apparatus according to the present invention.

The invention provides apparatus for treating an intact animal organism bearing a neoplastic process and undergoing drug-treatment for the neoplastic process, said apparatus comprising means of non-paramagnetic material for receiving an animal organism, means providing a magnetic circuit, said providing means being carried by said receiving means and including a multi-turn electrical coil, and means for generating electrical pulses for exciting said coil to generate a predetermined varying magnetic field whereby, in use, to generate, through electromagnetic induction, an electrochemical response in the cells and/or tissues of the animal organism, characterised in that the perimeter of the coil substantially encloses the receiving means, and in that the generating means is adapted to generate asymmetrical pulses, each being composed of first and second pulse portions, said first pulse portion having a first polarity and greater time duration and lesser magnitude than said second pulse portion, and said second pulse portion having a second polarity and lesser time duration and greater magnitude than said first pulse portion.

Such apparatus may be employed in aid of drug treatment by eliciting, via electromagnetic coupling, one or more specifically modified cell and/or tissue responses within the animal organism. Especially, the apparatus may assist in prolonging life-expectancy in a chemotherapy treatment of cancer, beyond the normal expectation from chemotherapy along, by altering the behaviour of living host and/or cancer cells to the benefit of the host, by electromagnetically inducing therein low-level voltage and concomitant current pulses of specific time-frequency-amplitude relations. Indeed, it has been found that, controlled application of excitation levels and repetition rates of such pulses a significant increase in life-expectancy is identifiable for cases involving drug treatment of cancer.

The invention will be illustratively described in conjunction with the accompanying drawings, in which:

Fig. 1 is a simplified view in perspective, to show electromagnetic apparatus of the invention, adapted for therapeutically beneficial operation upon one or more intact animal organisms afflicted with a neoplastic process;

Fig. 2 is a vertical sectional view through a midsection of the apparatus of Fig. 1;

Fig. 3 is an electrical block diagram schematically showing means for exciting the apparatus of Fig. 1;

Fig. 4 is a waveform diagram to illustrate operation of the apparatus when thus excited;

Fig. 5 is a computer-printed graph statistically presenting observed life-expectancy, involving use of the invention, on a comparative basis with non-use of the invention;

Fig. 6 is a simplified graphical display of tumor size as a function of time, the display being in aid of discussion of possible explanation for observed results.

Fig. 7 is a diagram to illuminate discussion of dual-coil placement considerations; and

Fig. 8 is a diagram similar to Fig. 7 to illustrate single-coil placement considerations; and

Figs. 9, 10 and 11 are simplified views in perspective to show further structural embodiments.

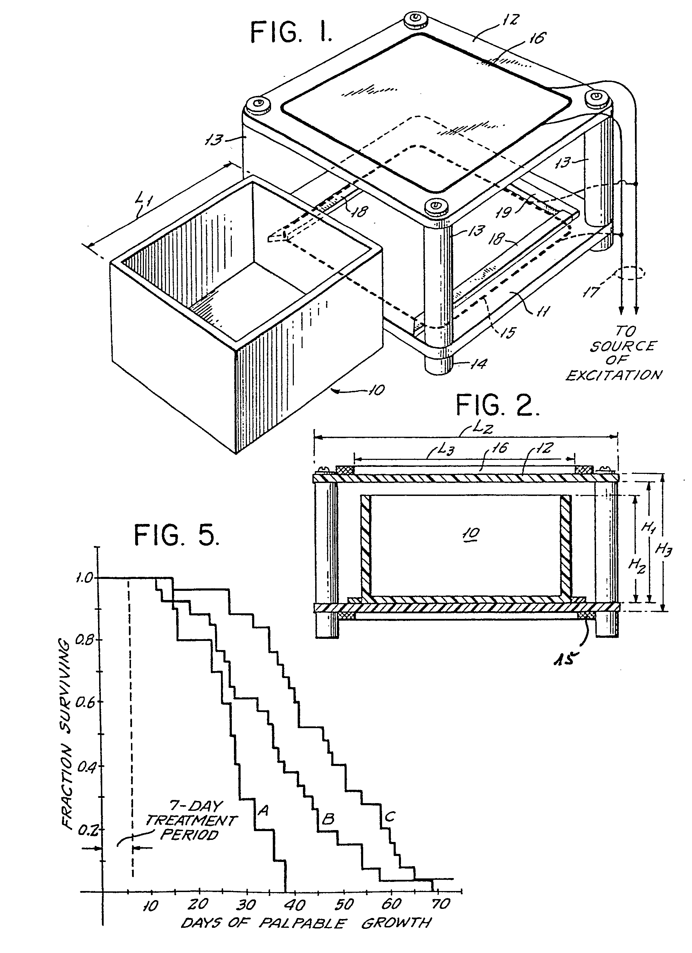

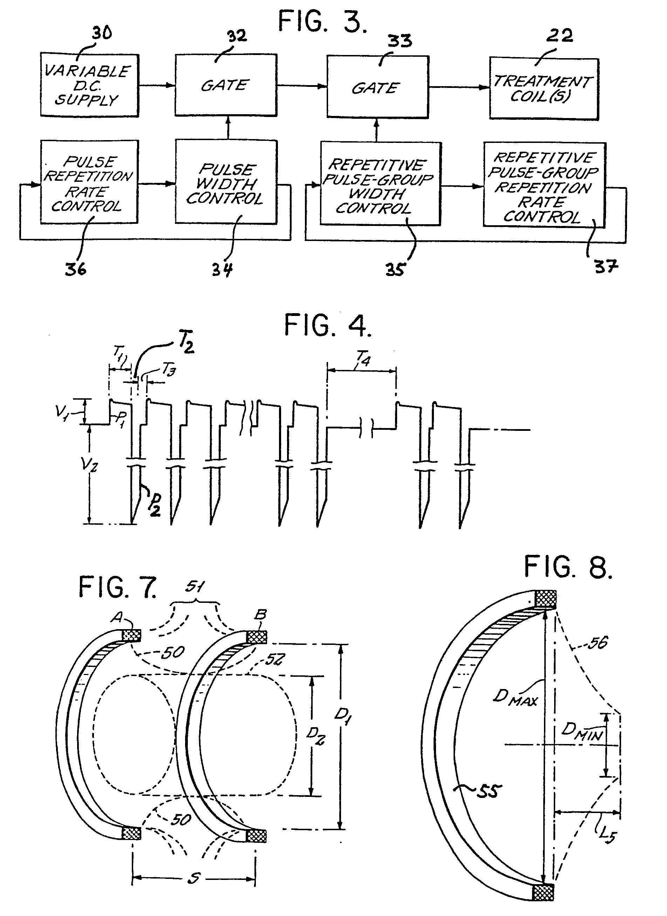

In Figs. 1 and 2, one or more intact animal organisms are housed within an open box-like container 10 of non-paramagnetic material, and the container is removably positionable upon a supporting shelf which is the lower one of two vertically spaced parallel plates 11—12 of non-paramagnetic material. Corner columns 13 rigidly space plates 11—12 to the extent $H_1$, which exceeds the height $H_2$ of container 10, to allow fresh-air access to contained animal organisms. Feet 14 establish an offset between the lower plate 11 and a supporting table (not shown), for protection of a lower electrical coil 15 that is secured to the underside of the lower plate 11. A duplicate electrical coil 16 is secured to the upper surface of the upper plate 12, in vertical registry with lower coil 15. Coils 15—16 are connected in flux-aiding relation, being shown parallel-connected and served by flexible-cable means 17 for supply by a source of excitation, to be described in connection with Fig. 3.

In the form shown, the container 10 is characterized by a square base and arrangement of sidewalls, of horizontal length $L_1$; and spaced guide rails 18 and a back stop 19 on the upper surface of plate 11 assure accurate removable central placement of container 10 on its shelf (plate 11) and with respect to the coils 15—16, when animal organisms within the container are exposed for treatment. Each of the plates 11—12 is square, of horizontal length $L_2$, and each of the coils 15—16 is essentially square, of inside horizontal length $L_3$. The volume of container 10 is preferably sized and positioned on shelf 11 so as to enclose all cells and/or tissues to be treated, within the geometrical volume defined by and between the coils 15—16; also, the spacing $H_3$ between coils 15—16 is preferably less than the effective diameter of the respective coils, as will later be explained in connection with Fig. 7. Since the particular coils 15—16 in Fig. 1 are square, their effective diameter may be approximated by the peripheral extent of the square $(4 \times L_3)$, divided by $\pi$.

Referring to Fig. 3, a variable d-c supply 30 is coupled through gate means 32 to the treatment coil 22 (or coils, as the case may be and as will more fully appear below); in conjunction with the apparatus of Fig. 1, the coil(s) 22 of Fig. 3 will be understood to symbolize connection to the line 17 which serves coils 15 and 16. The gate 32 is under the control of control units 34—36, which cause a pulse signal consisting of repetitive pulses of electrical potential to be applied to the coil means 22. Each pulse cycle, as shown in Fig. 4, is composed of a pulse portion $P_1$ of first polarity followed by a pulse portion $P_2$ of opposite polarity. The control units 34—36 may typically be monostable multivibrators, e.g., to generate appropriate timing signals, and they may be variable to control pulse duration and repetition rate within desired limits, while the use of a variable d-c supply 30 permits variation of the pulse-signal amplitude as desired.

With the pulse-generator apparatus thus far described, the output signal for coil excitation is in what has been described as Mode 1, being a steady succession of asymmetrical voltage pulses, the asymmetry being attributable to different time constants and being characterized first pulses $P_1$ of lesser amplitude $V_1$ and greater duration $T_1$, as compared with second pulses $P_2$ of greater amplitude $V_2$ and lesser duration $T_2$, there being a dwell $T_3$ between successive pulse cycles, depending upon circuit constants. The Mode 1 signal will produce cell-modulating effects, but we have thus far found a pulse-train (burst-segment) or Mode 2 excitation signal to be preferred for present purposes.

When pulse-train operation (Mode 2) is employed additional timing circuitry similar to units 34 and 36 is employed to define the burst-segment duration and the burst-segment repetition rate. As shown, control units 35—37 control gate 33 to produce a coil-excitation signal of the described asymmetrical nature, but wherein the control units 35—37 determine the number of pulses in a burst and the time $T_4$ between successive bursts of pulses.

It has been found that the signal across the treatment coil or coils, and hence the induced signal within cells and/or tissue under treatment, should satisfy certain criteria. These criteria will be specified with respect to the signal as induced in the cells under treatment. Such induced signal may be monitored, if desired, by use of an auxiliary monitoring probe or pickup coil (not shown) which is positioned at a distance from the coils A—B corresponding to the distance of the cells (and/or tissue) under treatment from such coils, as is explained in detail in said patent. It suffices here to state that a typical pickup coil that has been employed is circular, about one-half centimeter in diameter, and with about 67 or 68 turns of small gauge enamel-coated wire. The potential developed by the coil is divided by the length of the wire (in centimeters) to provide an induced voltage-per-centimeter number that is closely related to the volts/centimeter induced in the cells and/or tissue under treatment.

Example

In order to determine life-prolonging efficacy in application of the invention to an intact animal organism bearing a neoplastic process, ten identical structures as described in connection with Figs. 1 and 2 were identically prepared, each for containment of five $BDF_1$ mice, being a standard commercially available in-bred species. The mice came from a total collection of 80 $BDF_1$ females, 15 of which constituted a control group and were identically accommodated, in groups of five, in separate "dummy" containers, identical to the structure described in Figs. 1 and 2, except for the absence of coils 15—16. Care was taken that each container 10 provided identical housing, food supply and water-availability for the mice of all batches, the containers being lined with sawdust.

The containers 10 were 19 cm (7.5 inches) square ($L_1$), by 8.9 cm (3.5 inches) high ($H_2$), and plates 11—12 were 3.18 mm (1/8-inch) thick. The inside span ($L_3$) of the coils was 21.7 cm (8.5 inches), and they were spaced 10.2 cm (4 inches) ($H_3$). Each of the coils thus defined a 86.5 cm (34-inch) perimeter, having an effective diameter on the underside of 28 cm (11 inches), being defined by 20 turns of enamel-coated copper wire. Coil excitation with the circuit of Fig. 13 provided a probe-measured first polarity pulse amplitude $V_1$ of 0.16mv (in tissue) and duration $T_1$ of 250 $\mu$ sec., and a second-polarity pulse-amplitude $V_2$ of 1.6mv (in tissue) and duration $T_2$ of 4 $\mu$ sec., with a short dwell $T_3$ of about 4 $\mu$ sec. between pulses. Bursts of 50 milliseconds were administered concurrently to each electromagnetically treated batch, at a burst-repetition rate of 2 Hz, and the pattern was maintained for the same twelve hours of each day.

A test program was followed to determine the interaction of the described electromagnetic excitation with cytotoxic combination chemotherapy, and to determine such interaction in reference to such excitation alone (i.e. without chemotherapy) and in reference to the chemotherapy along (i.e. without electromagnetic excitation). All eighty mice were initially prepared with B16 melanoma, at a tumor diameter of 5.5 mm upon initiation of a seven-day period of different treatment and no-treatment circumstances as follows:

(a) The mice in three dummy containers, serving as "controls", were subjected to no chemotherapy and to no electromagnetic excitation.

(b) The mice in the remaining three dummy containers were subjected to combination chemotherapy, but to no electromagnetic excitation; the chemotherapy involved:

    (i) at 11.30 a.m., and on the first day only, an intraperitoneal injection of methylcyclohexylchloroethylnitroso-urea, 18 mg/kg, meaning that each mouse was weighed and given 18 mg of the drug, per kg of body weight, and

    (ii) at noon, of days 1 through 7, inclusive, a similar injection of cyclophosphamide, 12 mg/kg.

(c) The mice in the containers of five coil-equipped devices per Figs. 1 and 2 were subjected to electromagnetic excitation, but no chemotherapy; the electromagnetic excitation was pursuant to the above-described pulse-burst pattern, commencing at 12.30 p.m. on each of the seven days of chemotherapy treatment, and continuing for the next 12 hours of each day.*

_____
*In all cases involving electromagnetic-excitation therapy, the indicated pattern of excitation was repeated until all mice died, i.e. during and after the period of chemotherapy administration.

(d) The mice in the containers of the remaining five coil-equipped devices per Figs. 1 and 2 were subjected to both;

    (i) the combination chemotherapy of Item (b) above, and

    (ii) the electromagnetic-excitation therapy of Item (c) above.

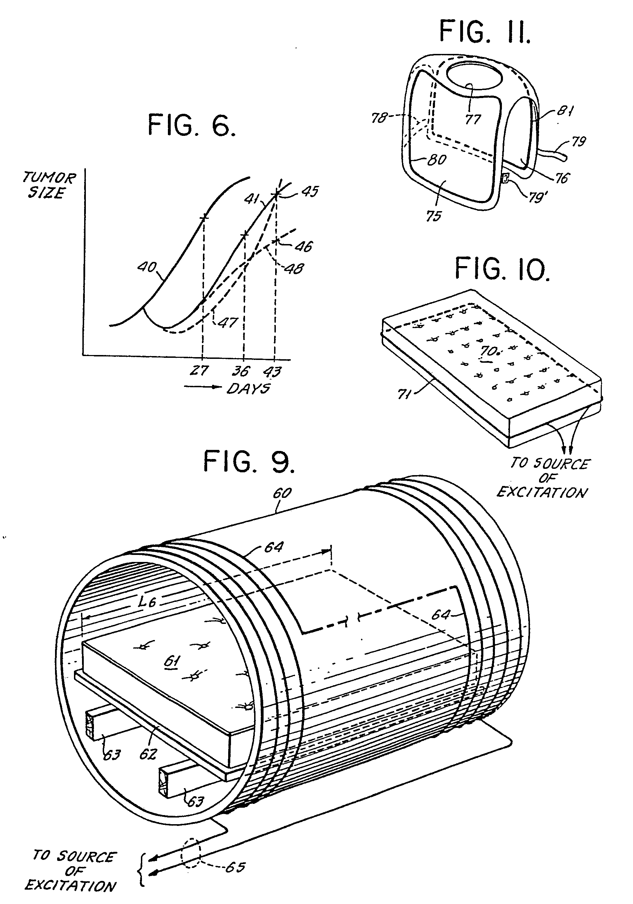

Taking initiation of the treatments as the origin of a time base, expressed in days of palpable growth, the observed results of the foregoing program are graphically presented in Fig. 5, in terms of the surviving fractional number of mice, at each applicable day. In Fig. 5, the curve A depicts observation of the "controls" group, Item (a) above; curve B depicts observation of the group receiving only combination chemotherapy, Item (b) above; and curve C depicts observation of the group receiving both combination chemotherapy and electromagnetic therapy, Item (d) above—it being noted that the group receiving only electromagnetic therapy was not observed to survive significantly different from the "controls" group, so that no curve in Fig. 5 appears for the group of Item (c) above. It is seen from the curves of Fig. 5 that the median survival of no-treatment "controls" mice was 27 days, that the median survival of mice treated with combination chemotherapy along was 36 days, and that the medial survival of mice treated with both combination chemotherapy and electromagnetic therapy was 45 days.

At the present time, it is not possible to account definitively for the observed extension of life-expectancy achieved through electromagnetic therapy in conjunction with chemotherapy, but Fig. 6 is a simplified presentation of growth curves which will aid a discussion of possible mechanisms that are operative. The solid-line curve 40 depicts the "controls" case, wherein the 27-day median-expectancy intercept is identified; and the solid-line curve 41 depicts the case of the combined chemotherapy alone, wherein the 36-day median-expectancy intercept is identified. The above-described experiment for the two cases conformed substantially to what had previously been established, as to both growth size and median expectancy; for this reason, these solid curves 40—41 provide a good base for discussion of possible mechanism to explain the newly discovered means of extension to 45 days. Of course, the fact that only 25 mice were subject to both the combined chemotherapy and the electromagnetic therapy, and the further observation that at death the tumor size was different for different animals, means that insufficient evidence exists with which to plot a median tumor size at death; thus in Fig. 6, two different size intercepts at 45 days are identified, at points 45 and 46, respectively.

First, it should be noted that the curve 41 for combined chemotherapy alone is generally parallel to the "controls" curve 40, at approximately 10-day offset therefrom, but that curve 41 begins as a perturbation characterized by downward departure from curve 40; this initial down slope reflects characteristic "regression" in the growth of the tumor cells (through killing tumor cells and possible augmentation of host response) and the subsequent rise in the curve reflects characteristic "regrowth" of the tumor. To account for a size denoted at point 45 for the observed 45-day expectancy, it is believed that addition of the electromagnetic therapy to the combined chemotherapy may produce a further offset of the regrowth curve, by reason of extended regression prior to regrowth; in other words, the electromagnetic therapy may so stimulate host cells as to enable the chemotherapy to have greater cell killing effectiveness. Such a situation is depicted by dashed-line curve 47.

It is also possible that the regrowth curve following such extended regression is not as steep as depicted by curve 47, in which case the slower rate of regrowth (not shown) may result in the lesser-size point 46 at the 45-day expectancy intercept.

Still further, the point 46 is shown in Fig. 6 to be identified with a lesser-slope regrowth curve 48, following the regression which is known to be identified with combination chemotherapy alone; in this event, it is reasoned that the electromagnetic therapy is operative to inhibit regrowth of the tumor, as by so coupling to the host body cells that they are better able to fight the regrowth.

Further in reference to Fig. 6, the point 45 is seen to indicate death at the 45-day intercept and for a tumor size considerably larger than what is to be expected (36-day intercept of curve 41) for the case of the chemotherapy alone. It is reasoned that in this event, the achievement of point 45, whether via curve 41 or via curve 47, may be attributable to the electromagnetic therapy so beneficially coupling to the host body cells that they increase the tolerance of the host body to the burden of a larger tumor.

Still further, where death may be attributed to toxity of the administered drugs, it is reasoned that the observed extended life-expectancy may be due to the electromagnetic therapy so beneficially coupling to the host body cells as to effectively decrease the toxicity of the drugs involved. And if this and/or any of other above-noted beneficial results of electromagnetic excitation of host body cells is correct, then it is further possible that such excitation of host body cells can reduce the metastatic potential of the tumor, i.e. reduce any capacity of the tumor to spread to non-contiguous regions of the host.

In the above-described structure and experimental example, it is considered important that the electromagnetic therapy shall involve exposure of entire animal organisms to substantially uniform and uniformly distributed (orientated) magnetic flux. This is for the reason that the indicated drug treatment necessarily spreads throughout the body, and it is believed that all host cells should be electromagnetically coupled to perform one or more of their beneficial functions, such as combatting tumor growth, increasing host resistance to drug toxicity, etc. The point as to substantially uniform and uniformly distributed (oriented) flux development will be briefly discussed in connection with the pair of circular coils A—B of Fig. 7.

On an elemental basis, it is convenient to consider the circular-coil situation depicted in Fig. 7, wherein like circular coils A—B of inside diameter $D_1$ are positioned on a common central axis of symmetry, at parallel planes which are spaced apart by the distance S, and wherein the coils A—B are excited in flux-aiding relation. If the spacing S is sufficiently small in relation to the diameter $D_1$, then substantially all flux lines within coils A—B will extend continuously therebetween, on a generally straight alignment which may even neck down, as suggested by the profile 50. If the spacing S is greater (again in relation to the diameter $D_1$), some stray-flux lines 51 will develop, to the detriment of the development of uniform high-density flux in the central span S. Generally, in view of the necking down (50), and in view of the treatment zone being generally at the centre span S, it is convenient to consider the coils A—B as being desirably effective in producing the uniform flux distribution over an imaginary cylinder 52 of diameter $D_2$, tangent to the neck-down profile 50. From experience to date, it can be stated that for body-cell application of the character presently described, the span S should be equal to or less than the diameter $D_1$, and of course $D_2$ (the effective diameter of the zone of body-cell treatment) will always be considerably less than $D_1$, being substantially equal to $D_1$ only when coils A—B are closely adjacent. As a practical consideration in the application of dual coils to an animal organism specimen or batch, it is believed that the nominal inside diameter $D_1$ of the coils should be at least 1.5 times the diameter $D_2$ of the effective cell-treatment zone, and this has been found to be a reliable approach for coil spacing S substantially equal to the inside diameter $D_1$.

Further applications of the invention will be indicated in discussion of the successive embodiments of Figs. 8 to 10.

In Fig. 8, a large simple multiturn coil 55 is shown, in the schematic style adopted for the discussion of the two spaced coils A—B of Fig. 7. The coil 55 happens to be shown as circular, but this is only for purposes of simplified discussion of principle. The coil 55 is shown to have an inside diameter $D_{max.}$, establishing essentially a central plane (the plane of the coil) through which all flux lines must be confined; in response to coil excitation at this plane, flux distribution is essentially uniform and orientated perpendicular to the plane, i.e., parallel to the central axis of the coil. Outside this plane, the lines of the magnetic field bloom axially and radically outwardly, to develop generally toroidally about the coil; but, at least for a shallow axial offset, of extent $L_5$, a region exists wherein the orientation and distribution of magnetic flux is useful for the therapeutic purposes of the invention. The geometrical solid within which such useful flux is available is schematically indicated by the generally conically tapering contour 56, reducing from $D_{max.}$ at the central plane of the coil 55, to a minimum useful diameter $D_{min.}$ at the offset $L_5$. The single multiturn coil 55, excited as described for the circuit of Fig. 3, may have utility in treatment of tumors which by their nature are essentially confined near a surface locale of the animal organism to be treated. Of course, the size and placement of the coil 55 on the body should be such that the afflicted region is contained within the offset geometrical volume between contour 56 and the plane of the coil. Utility also exists for an arrangement as in Fig. 10, discussed below.

Fig. 9 illustrates apparatus useful in applying electromagnetic therapy to an entire human body and is thus indicated for use with chemotherapy, analogous to the situation in the foregoing illustrative example. A large tubular shell 60 extends longitudinally for essentially the length $L_6$ of a bed mattress 61 which, with its supporting platform 62 is fully contained within the lower semi-cylindrical half of the volume within the shell 60. Longitudinal frame members 63 provide means of stabilizing support for platform 62 with respect to the enclosure of shell 60, and will be understood also to derive floo-based reference from suitable outer frame structure (not shown); it will be further understood that all structural elements 60, 61, 62, 63 are of non-paramagnetic materials. Importantly, electrical-coil means, which may be uniformly spaced turns of a single coil 64, are mounted to and developed along the cylindrical periphery of shell 60, with a lead-connection cable 65 to a suitable source of excitation, exemplified by the circuit of Fig. 3. Since the spacing between individual turns of coil 64 more than amply meets the spacing (S) vs. diameter ($D_1$) criteria of Fig. 7, the excitation of coil 64 necessarily establishes uniform and well-orientated flux within shell 60, for cell and/or tissue coupling to the entire body of the patient accommodated at 61. With the apparatus of Fig. 9, it is thus possible to assure full electromagnetic body-cell response as may be needed in conjunction with chemotherapy.

In connection with Fig. 9, the frame members will be understood to suggest frame-based elongate guide rails, extendable beyond one of the ends of shell 60, for convenient guided insertion of the patient, the mattress 61 and its platform 62, into the electromagnetic treatment zone within shell 60.

Fig. 10 shows a simpler structure for providing full human-body exposure to an electro-magnetic-treatment zone, but wherein manifestly, the totality of such exposure cannot be assured to the extent provided by the apparatus of Fig. 9. In Fig. 10 a complete mattress 70 of non-paramagnetic material peripherally carried a single multiturn coil 71 which may be embodied in one of the welts of the mattress but which is shown intermediate the upper and lower welts, for protection of coil-winding insulation and for mattress reversability. The flux uniformity and orientation considerations discussed in general in connection with Fig. 8 apply also to Fig. 10, so that the patient who rests on mattress 70 may be virtually assured of adequate magnetic-flux exposure when coil 71 is excited, as long as he remains properly central of the mattress. Strap devices (not shown) exist for use with the mattress to confine the patient as desired on the mattress 70.

Fig. 11 shows application of a two-coil system to a supporting garment or vest having front and back fabric panels 75—76 which are connected over an upper shoulder region having a central opening 77, for head and neck accommodation. A tie such as an elastic band 78 connects panels 75—76 on one side, and a hooked tie 79 detachably connects panels 75—76 on the other side. A generally square first coil 80 maximizes use of available front-panel area, and a similar coil 81 is similarly carried by the rear panel 76. Although not thus shown, it will be understood that coils 80—81 are connected in flux-aiding relation, and simple detachable provision in the vicinity of and symbolized by fastening means 79' will serve for flexible and selective electrical connection of coils 80—81 to their source of excitation.

A full and complete understanding of the mechanism by which cell and/or tissue behavior is modified by the invention has yet to be given. It is presently believed, however, that each cell importantly includes an outer layer which is relatively electrically conductive, thus establishing for each cell what may be viewed as a short-circuited single-turn secondary, inherently susceptible to voltage induction and therefore voltage generation within each cell that is exposed to the time-varying magnetic field established by the particular excited treatment coil or coils. It is believed that each cell may exhibit non-linear electrical response and/or (non-linear electrochemical response) to cell-induced voltage in the presence of a low-level time-varying magnetic field. Thus, non-linearity in the response of the cell itself may perhaps be as much responsible as any other factor in achieving the described results.

For a low-level time-varying electrical signal of arbitrary waveform to achieve a net biological effect, it is believed that the receiver (the host cell and/or tissue, and/or the cancer cells) must possess non-linear characteristics. For example, if a net perturbation (i.e., net, over a period of time) of the amount of a specifically adsorbed (bound) ion at a cell-membrane site is the target or goal, then the use of induced current can cause this to occur only if the process exhibits a certain degree of irreversibility (i.e., non-linearity); in the case of body-cell stimulation, it is believed that activation of calcium-binding protein may be specifically involved; in addition, it appears that adsorption and/or transmembrane passage of cations (e.g., $Ca^{++}$, $Na^+$, $K^+$) are also believed to be significant to the response. This non-linear property is also coupled with the basic kinetics (relaxation time) of the process. In other words, the parameters of the perturbing signal must be such that efficient coupling to the biological cell process can be achieved without such excessive amplitude as would remove the signal from the low-level category and would invite the occurrence of other non-electrical effects, such as thermal and ionizing effects, which it will be recalled from the present specific example are not measurably present for the low-level amplitude example given.

Quantitative assessment of the manner by which informational signals couple to cellular processes having the just described characteristics can be achieved by considering that (i) a surface process is involved, (ii) its basic kinetics are exponential in nature, (iii) net response during each cycle of a signal of arbitrary waveshape is given by a generalized expression for P, the net perturbation occurring for the duration of the waveform (i.e., $T_1+T_2$), (iv) the various repetitive natures of the signal burst and/or repetition rate, as well as the basic waveform-timing characteristics can be conveniently quantitated via spectral analysis using Laplace transformation, and (v) the overall net perturbation P can then be described in terms of its frequency content in the context of the electrical waveform characteristics.

In the above manner, the desirable characteristics of a waveform of any shape can be chosen to provide a correlation between its frequency spectrum (as determined by the electrochemical cell-surface process) and the elicited biological response. Generally, there will be an amplitude range (or ranges) over which the system receives an adequate "dose"-to-modulate function. The number of specific ranges depends upon system complexity. In other words, the more biological stages having distinct control processes, the more amplitude ranges may be present for selective operation upon the different control processes.

For example, in spite of the low level indicated for the present specific example of electromagnetic excitation on an intact animal organism bearing a neoplastic process, there is ample reason to believe that still lower or higher levels (e.g., lower to the extent of an order of magnitude or more) may be equally if not more effective in extending life-expectancy or otherwise improving upon one or more of the current approaches to drug treatment of solid tumors and/or leukemia.

It is also to be understood that in view of the incomplete and perhaps incorrect nature of one or more of the foregoing explanations for tumorous body response to drug treatment concurrent with electromagnetic treatment, the invention is not to be deemed limited by such explanations. And it is further to be understood that the invention may also have application to such recognized techniques of drug treatment of neoplastic processes (other than chemotherapy) as immunotherapy, hormone therapy and interferon therapy. Also, in the present context of incompletely understanding the precise effect of electromagnetic therapy in conjunction with drug treatment of cancer, the possibility also exists that the described electromagnetic coupling may significantly alter drug metabolism, pharmacology or distribution in the body.

## Claims

1. Apparatus for treating an intact animal organism bearing a neoplastic process and undergoing drug-treatment for the neoplastic process, said apparatus comprising means (10, 11, 12; 60, 61, 62, 63; 70; 75, 76) of non-paramagnetic material for receiving an animal organism, means (15, 16; 64; 71; 80, 81) providing a magnetic circuit, said providing means being carried by said receiving means and including a multi-turn electrical coil and means for generating electrical pulses for exciting said coil to generate a predetermined varying magnetic field whereby, in use, to generate, through electromagnetic induction, an electrochemical response in the cells and/or tissues of the animal organism, characterised in that the perimeter of the coil substantially encloses the receiving means, and in that the generating means is adapted to generate asymmetrical pulses, each being composed of first and second pulse portions, said first pulse portion (P'1) having a first polarity and greater time duration and lesser magnitude than said second pulse portion (P'2), and said second pulse portion having a second polarity and lesser time duration and greater magnitude than said first pulse portion.

2. Apparatus according to claim 1, characterised in that said receiving means (10, 11, 12; 60, 61, 62, 63; 70) has a horizontal support surface for supporting the entire animal organism.

3. Apparatus according to claim 2, characterised in that said coil is one of two vertically spaced and axially aligned coils (15, 16) having said support surface therebetween.

4. Apparatus according to claim 3, characterised in that the spacing between said coils (15, 16) is less than the effective internal diameter of said coils.

5. Apparatus according to claim 2, characterised in that said coil (64) is helical and extends longitudinally of said support surface.

6. Apparatus according to claim 5, characterised in that said receiving means includes an elongate tubular shell (60) of non-paramagnetic material supporting said coil.

7. Apparatus according to claims 2, 5 or 6, characterised in that said receiving means includes a mattress (61) of standard length for accommodation of an adult human.

8. Apparatus according to claim 1, characterised in that said receiving means (75, 76) comprises an article of clothing.

9. Apparatus according to claim 8, characterised in that said article of clothing comprises a body vest having front and back panels (75, 76) for application to the front and back of a human body to be treated, said coil being one of two coils (80, 81) secured to the respective front and back panels and connected in flux aiding relation.

10. Apparatus according to claim 9, characterised in that the effective diameter of said coils is greater than their axial spacing when the body vest is worn on the body.

11. Apparatus according to any preceding claims, characterised in that said generating means are adapted to generate said pulses in a limited burst.

12. Apparatus according to claim 11, characterised in that the burst is repeated at a predetermined burst repetition rate.

## Patentansprüche

1. Vorrichtung zur Behandlung eines intakten Tierorganismus, der an einem neoplastischen Prozeß leidet und einer Arzneimittelbehandlung gegen diesen Prozeß unterliegt, mit einer Einrichtung (10, 11, 12; 60, 61, 62, 63; 70; 75, 76) aus nicht-paramagnetischem Material zur Aufnahme eines Tierorganismus, einer Einrichtung (15, 16; 64; 71; 80, 81) zur Herstellung eines magnetischen Kreises, die von der Aufnahmeeinrichtung getragen wird und eine elektrische Mehrfachwicklungsspule umfaßt, und mit einer Einrichtung zur Erzeugung elektrischer Impulse zur Erregung der Spule, um ein bestimmtes variierendes magnetisches Feld zu erzeugen, wodurch im Betrieb mittels elektromagnetischer Induktion eine elektrochemische Reaktion in den Zellen und/oder Zellgeweben des Tierorganismus hervorgerufen wird, dadurch gekennzeichnet, daß der Umfang der Spule im wesentlichen die Aufnahmeeinrichtung umschließt, und daß die Erzeugereinrichtung asymmetrische Impulse erzeugen kann, die jeweils aus einem ersten und einem zweiten Impulsabschnitt zusammengesetzt sind, wobei der erste Impulsabschnitt (P'1) eine erste Polarität und größere Zeitdauer sowie geringere Größe als der zweite Impulsabschnitt (P'2) hat, und wobei der zweite Impulsabschnitt eine zweite Polarität und geringere Zeitdauer sowie eine größere Höhe als der erste Impulsabschnitt hat.

2. Vorrichtung nach Anspruch 1, dadurch gekennzeichnet, daß die Aufnahmeeinrichtung (10, 11, 12; 60, 61, 62, 63; 70) eine waagrechte Tragfläche zum Tragen des gesamten Tierorganismus hat.

3. Vorrichtung nach Anspruch 2, dadurch gekennzeichnet, daß die Spule eine von zwei vertikal beabstandeten und axial ausgerichteten Spulen (15, 16) ist, zwischen denen die Tragfläche angordnet ist.

4. Vorrichtung nach Anspruch 3, dadurch gekennzeichnet, daß der Abstand zwischen den Spulen (15, 16) geringer als der wirksame innere Durchmesser der Spulen ist.

5. Vorrichtung nach Anspruch 2, dadurch gekennzeichnet, daß die Spule (64) schneckenförmig ist und sich längs der Tragfläche erstreckt.

6. Vorrichtung nach Anspruch 5, dadurch gekennzeichnet, daß die Aufnahmeeinrichtung ein längliches rohrförmiges Gehäuse (60) aus nicht-paramagnetischem Material umfaßt, das die Spule trägt.

7. Vorrichtung nach Anspruch 2, 5 oder 6, dadurch gekennzeichnet, daß die Aufnahmeeinrichtung eine Matraze (61) in Standardlänge zur Aufnahme eines erwachsenen Menschen umfaßt.

8. Vorrichtung nach Anspruch 1, dadurch gekennzeichnet, daß die Aufnahmeeinrichtung (75, 76) ein Bekleidungsstück umfaßt.

9. Vorrichtung nach Anspruch 8, dadurch gekennzeichnet, daß das Bekleidungsstück ein Körpergewand mit Vorder- und Rückseiten (75, 76) zum Auflegen auf die Vorder- und Rückseite eines zu behandelnden menschlichen Körpers umfaßt, wobei die Spule eine von zwei Spulen (80, 81) ist, die an den jeweiligen Vorder- und Rückseiten befestigt und in flußunterstützender Beziehung verbunden sind.

10. Vorrichtung nach Anspruch 9, dadurch gekennzeichnet, daß der wirksame Durchmesser der Spulen größer als ihr axialer Abstand ist, wenn das Körpergewand auf dem Körper getragen ist.

11. Vorrichtung nach einem der vorangehenden Ansprüche, dadurch gekennzeichnet, daß die Erzeugereinrichtung Impulse in der Form begrenzter Impulsbündel (Burst) erzeugen kann.

12. Vorrichtung nach Anspruch 11, dadurch gekennzeichnet, daß die Impulsbündel in einer bestimmten Impulsbündel-Wiederholungsrate wiederholt werden.

## Revendications

1. Appareil pour le traitement d'un organisme animal intact porteur d'un processus néoplasique et soumis à un traitement par médicaments du processus néoplasique, ledit appareil comportant un moyen (10, 11, 12; 60, 61, 62, 63; 70; 75, 76) en un matériau non-paramagnétique pour recevoir un organisme animal, des moyens (15, 16; 64; 71; 80, 81) constituant un circuit magnétique, ces derniers moyens étant portés par ledit moyen de réception et comportant une bobine électrique à plusieurs spires, et un moyen de génération d'impulsions électriques pour l'excitation de ladite bobine afin de générer un champ magnétique variable prédéterminé par lequel, en utilisation, est engendrée, par induction électromagnétique, une réponse électrochimique dans les cellules et/ou les tissus de l'organisme animal, caractérisé en ce que le périmètre de la bobine entoure sensiblement le moyen de réception, et en ce que le moyen de génération est adapté à générer des impulsions asymétriques dont chacune comporte une première et deuxième parties d'impulsion, ladite première partie d'impulsion (P'1) possédant une première polarité ainsi qu'une durée de temps plus grande et une amplitude plus petite que ladite deuxième partie d'impulsion (P'2) et ladite deuxième partie d'impulsion possédant une deuxième polarité, ainsi qu'une durée de temps moindre et une amplitude plus grande que ladite première partie d'impulsion.

2. Appareil selon la revendication 1, caractérisé en ce que le moyen de réception (10, 11, 12; 60, 61, 62, 63; 70) a une surface horizontale d'appui pour supporter l'organisme animal tout entier.

3. Appareil selon la revendication 2, caractérisé en que que ladite bobine est l'une des bobines d'un groupe de deux bobines espacées verticalement et alignées sur le même axe (15, 16), ladite surface d'appui étant disposée entre ce deux bobines.

4. Appareil selon la revendication 3, caractérisé en ce que la longueur de l'espace entre lesdites bobines (15, 16) est inférieure au diamètre effectif intérieur desdites bobines.

5. Appareil selon la revendication 2, caractérisé en ce que ladite bobine (64) est hélicoïdale et s'étend longitudinalement sur ladite surface d'appui.

6. Appareil selon la revendication 5, caractérisé en ce ledit moyen de réception comporte une coquille tubulaire allongée (60) en un matériau non paramagnétique, supportant ladite bobine.

7. Appareil selon les revendications 2, 5 ou 6, caractérisé en ce que ledit moyen de réception comprend un matelas (61) de longueur standard pour recevoir un être humain adulte.

8. Appareil selon la revendication 1, caractérisé en ce que ledit moyen de réception (75, 76) comprend un vêtement.

9. Appareil selon la revendication 8, caractérisé en ce que ledit vêtement comprend un tricot de corps possédant des panneaux de devant et de derrière (75, 76) pour l'application du devant et du dos d'un corps humain à traiter, ladite bobine étant l'une des bobines d'un groupe de deux bobines (80, 81) fixées respectivement sur les panneaux de devant et de derrière et branchées dans le sens d'une addition des flux.

10. Appareil selon la revendication 9, caractérisé en ce que le diamètre effectif desdites bobines est plus grand que leur espacement axial lorsque le tricot de corps est en place sur le corps.

11. Appareil selon l'une quelconque des revendications précédentes, caractérisé en ce que ledit moyen de génération est adapté pour générer lesdites impulsions en une rafale limitée.

12. Appareil selon la revendication 11, caractérisé en ce que la rafale est répetée à un taux de répétition de rafale prédéterminé.

FIG. 1.

TO SOURCE OF EXCITATION

FIG. 2.

FIG. 5.

7-DAY TREATMENT PERIOD

FRACTION SURVIVING

DAYS OF PALPABLE GROWTH

# FIG. 3.

# FIG. 4.

# FIG. 7.

# FIG. 8.

0 039 988

FIG. 11.

FIG. 6.

TUMOR SIZE

DAYS

FIG. 10.

FIG. 9.

TO SOURCE OF EXCITATION

TO SOURCE OF EXCITATION

3